# EUROPÄISCHE PATENTSCHRIFT

(11) **EP 0 799 237 B1**
(45) Veröffentlichungstag und Bekanntmachung des Hinweises auf die Patenterteilung: **12.05.1999**
(21) Anmeldenummer: 95941668.6
(22) Anmeldetag: 05.12.1995
(51) Int. Cl.: C07H 23/00, A61K 31/70, A61K 35/78

(54) **VITAMIN B 12-HALTIGE SANDDORNKONZENTRATE ODER -EXTRAKTE**
VITAMIN B 12-CONTAINING SALLOW THORN CONCENTRATES OR EXTRACTS
CONCENTRES OU EXTRAITS D'ARGOUSIER CONTENANT DE LA VITAMINE B 12

(30) Priorität: 22.12.1994 DE 4446092
(43) Veröffentlichungstag der Anmeldung: 08.10.1997
(73) Patentinhaber: PANDALIS, Georgios, D-49219 Glandorf (DE)
(72) Erfinder: PANDALIS, Georgios, D-49219 Glandorf (DE)
(74) Vertreter: Grünecker, Kinkeldey, Stockmair & Schwanhäusser Anwaltssozietät
(86) Internationale Anmeldenummer: EP9504776
(87) Internationale Veröffentlichungsnummer: WO9619490

(56) Entgegenhaltungen:
- DATABASE WPI Section Ch, Week 8636 Derwent Publications Ltd., London, GB; Class B04, AN 237593 XP002002868 & SU,A,1 207 473 (PYATIGOR PHARM. INST.) , 30.Januar 1986
- DATABASE WPI Section Ch, Week 9504 Derwent Publications Ltd., London, GB; Class B04, AN 028939 XP002002869 & RU,A,2 011 383 (PYATIGORSK PHARM. INST.) , 30.April 1994
- DATABASE WPI Section Ch, Week 9523 Derwent Publications Ltd., London, GB; Class D23, AN 176630 XP002002870 & RU,A,2 019 991 (KALININ POLY) , 30.September 1994
- PATENT ABSTRACTS OF JAPAN vol. 14, no. 14 (C-674), 12.Januar 1990 & JP,A,01 125748 (SHIRO NAGAI), 13.Oktober 1989,
- DATABASE WPI Section Ch, Week 9110 Derwent Publications Ltd., London, GB; Class B02, AN 069066 XP002002871 & JP,A,03 017 095 (JAPAN ORGANO KK) , 25.Januar 1991

## Beschreibung

Die vorliegende Erfindung betrifft Vitamin B₁₂-haltige Sanddornkonzentrate oder -extrakte, Verfahren zu deren Herstellung und die Verwendung von Sanddorn.

Vitamine der Vitamin B₁₂-Gruppe sind die halbsystematisch Cobalamine genannten Cobalt(III)-haltigen Corrinoide. Unter Vitamin B₁₂ schlechthin versteht man im allgemeinen das Cyanocobalamin, das bei der Isolierung aus dem natürlich vorkommenden Coenzym B₁₂ in Gegenwart von Cyanid-Ionen entsteht. Vitamin B₁₂ gehört neben Biotin zu den Spurenvitaminen. Eine ausreichende Vitamin B₁₂-Zufuhr ist vor allem für die normale Blutbildung (Erythropoese) erforderlich, außerdem für die Funktion der Nervenzellen und für das Wachstum. Insbesondere stimuliert es die Nukleinsäuresynthese. Bei einem Mangel an Vitamin B₁₂ ist die Entwicklung der Erythrozyten im Knochenmark gestört (Römpp Chemie Lexikon, 9. Auflage, 1992, Seite 4946).

Mangelzustände an Vitamin B₁₂ können bedingt sein durch Endoparasitenbefall (z.B. Fischbandwurm), chronische Magen-Darm-Erkrankungen, Einnahme von Medikamenten (z.B. bestimmte Antikonvulsiva oder orale Antidiabetika) und bei chronischer Unterversorgung, insbesondere bei Meidung tierischer Lebensmittel bei streng vegetarischer Ernährung (Teufel, Ternes, Tunger, Zobel, Lebensmittellexikon, Band 2, 3. Auflage, 1993, Seite 796).

Mikroorganismen sind die bisher einzig bekannten Lebewesen, die Vitamin B₁₂ synthetisieren können. Somit wird bei verschiedenen Tierarten über die enterale Synthese (Darmflora) ein mehr oder weniger entscheidender Beitrag zur Bedarfsdeckung geleistet. Bei vielen Tieren (Herbivoren) reicht die enterale Eigensynthese völlig aus, Carnivoren decken ihren Bedarf nicht nur über die Synthese durch die Darmflora, sondern gleichzeitig durch die Vitamin B₁₂-Aufnahmen mit Fleisch. Der Mensch kann enteral, d.h. im Dickdarm synthetisiertes, Vitamin B₁₂ nur unzureichend ausnutzen und ist deshalb auf die zusätzliche Aufnahme von Vitamin B₁₂ mit der Nahrung angewiesen. Wesentliche Vitamin B₁₂-Quellen, die zur Bedarfsdeckung beitragen, sind tierische Produkte, vor allem Leber, Niere, Herz, aber auch Eier und Milch. Rein vegetarische Kost ist nahezu frei von Vitamin B₁₂. (K.-H. Bässler, E. Grühn, D. Loew und H. Petrzik, Vitaminlexikon, Gustav Fischer Verlag, Stuttgart, Jena, New York, 1992, Seite 96).

Als Tagesdosis an Vitamin B₁₂ werden für Erwachsene von der deutschen Gesellschaft für Ernährung 5 µg empfohlen.

Von Sanddorn (Hippophaë rhamnoides) ist bekannt, daß insbesondere die Früchte reich an Vitamin C und anderen Inhaltsstoffen sind. Die wichtigsten Inhaltsstoffe des Sanddorns sind einige Fruchtsäuren, für Obst ungewöhnlich viel Fett, überdurchschnittlich viel Calcium und Magnesium und vor allem Provitamin A und die Vitamine E, B₁, B₂, B₈ und C. Von letzterem werden extrem hohe Werte erreicht, die abhängig vom Standort 200 bis 1300 mg pro 100 g betragen. Sanddorn wird deshalb zur Vitaminisierung von Nähr- und Kräftigungsmitteln, Konserven und Suppenwürzen sowie von Arzneimitteln verwendet (G. Liebster, Warenkunde Obst und Gemüse, Morion-Verlag, 1988, Seite 221 ff).

Aufgabe der vorliegenden Erfindung ist es, ein neues Vitamin B₁₂-haltiges Mittel auf rein pflanzlicher Basis und ein Verfahren zu seiner Herstellung zur Verfügung zu stellen.

Die Aufgabe wird erfindungsgemäß durch Vitamin B₁₂-haltige Sanddornkonzentrate oder -extrakte, sowie Verfahren zu deren Herstellung gelöst. Das Verfahren zur Herstellung des Vitamin B₁₂-haltigen Sanddornkonzentrats ist dadurch gekennzeichnet, daß man aus Sanddorn die darin enthaltenen flüssigen und/oder flüchtigen Bestandteile abtrennt. Das Verfahren zur Herstellung des Vitamin B₁₂-haltigen Sanddornextrakts ist dadurch gekennzeichnet, daß man Vitamin B_{12,} gegebenenfalls zusammen mit anderen Inhaltsstoffen, in an sich bekannter Weise aus Sanddorn extrahiert.

Erfindungsgemäß können sämtliche Pflanzenteile, d.h. die Früchte, Blätter, Stengel, Wurzeln und Blüten von Sanddorn, eingesetzt werden; insbesondere werden die Sanddornfrüchte verwendet.

Die in Sanddorn enthaltenen flüssigen und/oder flüchtigen Bestandteile, insbesondere Wasser, können aus dem Sanddorn durch übliche Maßnahmen, beispielsweise durch Trocknung mit Luft, insbesondere mit vorgetrockneter Luft, Trocknung durch überkritisches CO₂, Gefriertrocknen, Sprühtrocknen oder Einengen im Vakuum, abgetrennt werden. Das Konzentrierungsverhältnis liegt vorzugsweise im Bereich von 1:1 bis 1:20, insbesondere im Bereich von 1:5 bis 1:15.

Das Vitamin B₁₂ kann, gegebenenfalls zusammen mit anderen Inhaltsstoffen, aus dem Sanddorn mittels üblicher Extraktionsverfahren gewonnen und anschließend vorzugsweise aufkonzentriert werden. Das Aufkonzentrieren kann mittels der vorstehend beschriebenen Verfahren durchgeführt werden, wodurch man vorzugsweise dickflüssige, pastöse, pulverförmige oder kristalline Extrakte erhält. Extraktionsverfahren, die erfindungsgemäß angewendet werden können, umfassen kontinuierliche und diskontinuierliche Verfahren, insbesondere Flüssig-Flüssig-Extraktionsverfahren. Als Lösungsmittel können sowohl anorganische als auch organische, polare und unpolare Lösungsmittel eingesetzt werden, abhängig davon, welche Inhaltsstoffe des Sanddorns außer Vitamin B₁₂ extrahiert werden sollen. Bevorzugte Extraktionsmittel für Vitamin B₁₂ sind aliphatische Kohlenwasserstoffe, insbesondere Hexan, überkritisches CO₂, Wasser und niedere Alkohole mit 1-5 C-Atomen, insbesondere Ethanol. Es ist auch möglich, verschiedene Inhaltsstoffe mit unterschiedlichen Lösungsmitteln zu extrahieren und die Extrakte anschließend zu vereinigen.

Sanddornextrakte werden vorzugsweise durch Ausschütteln, Auslaugen oder Auskochen des Sanddorns oder durch Extraktionsverfahren, wie Gleichstrom-, Gegenstrom- oder Kreuzstrom-Extraktion, sowie durch Tropfen-Gegenstrom-Chromatographie, hergestellt.

Überraschend wurde gefunden, daß Sanddorn neben den oben genannten Vitaminen und Mineralstoffen auch eine hohe Menge an Vitamin B₁₂ enthält. Der Gehalt an Vitamin B₁₂ in Sanddorn wurde mittels kompetitiven Enzymimmunoassay ermittelt. Dieses Bestimmungsverfahren ist in Deutsche Lebensmittel-Rundschau, 86. Jahrgang, Heft 10, 1990, Seite 307-310 beschrieben. Für getrocknete Sanddornbeeren wurde ein Wert 32 pg Vitamin B₁₂/100 g nach diesem Verfahren ermittelt.

Dieser Wert ist vergleichbar mit den Vitamin B₁₂-Gehalten von Schweineleber mit 25-39 µg/100 g und Rinderniere mit 31-33 µg/100 g, während Nahrungsmittel, wie Milch mit etwa 0,4 µg/100 g und Eier mit 2,5-3 µg/100 g, nur eine relativ geringe Menge an Vitamin B₁₂ enthalten.

Sanddorn kann somit erfindungsgemäß erfolgreich zur Behandlung oder Prophylaxe von Vitamin B12-Mangelerscheinungen verwendet werden. Insbesondere eignet er sich zur Behandlung von Neuropathien und/oder Erythropoesestörungen und/oder zur Stimulation der Nukleinsäuresynthese.

Durch Sanddorn als Vitamin B₁₂-Quelle wird erstmals auch reinen Vegetariern die Möglichkeit gegeben, ihren Vitamin B₁₂-Bedarf aus pflanzlichen Produkten zu decken. Dies betrifft auch Personen, bei denen Unverträglichkeiten mit tierischen Eiweißen zu einer Unterversorgung mit Vitamin B₁₂ führen.

## Patentansprüche

1. Vitamin B₁₂-haltige Sanddornkonzentrate oder -extrakte.

2. Verfahren zur Herstellung eines Vitamin B₁₂-haltigen Sanddornkonzentrats, dadurch gekennzeichnet, daß man aus Sanddorn die darin enthaltenen flüssigen und/oder flüchtigen Bestandteile abtrennt.

3. Verfahren nach Anspruch 2, dadurch gekennzeichnet, daß man die flüssigen und/oder flüchtigen Bestandteile durch Trocknung mit Luft, Trocknung durch überkritisches CO₂, Gefriertrocknen, Sprühtrocknen oder Einengen im Vakuum abtrennt.

4. Verfahren zur Herstellung eines Vitamin B₁₂-haltigen Sanddornextrakts, dadurch gekennzeichnet, daß man Vitamin B₁₂, gegebenenfalls zusammen mit anderen Inhaltsstoffen, in an sich bekannter Weise aus Sanddorn extrahiert.

5. Verfahren nach Anspruch 4, dadurch gekennzeichnet, daß man eine kontinuierliche oder diskontinuierliche Flüssig-Flüssig-Extraktion durchführt.

6. Verfahren nach einem der Ansprüche 2 bis 5, dadurch gekennzeichnet, daß man Sanddornfrüchte als Ausgangsmaterialien einsetzt.

7. Verwendung von Sanddorn zur Herstellung eines Medikamentes zur Prophylaxe oder Behandlung von Vitamin B12-Mangelerscheinungen.

8. Verwendung nach Anspruch 7 zur Herstellung eines Medikamentes zur Behandlung von Neuropathien und/oder Erythropoesestörungen und/oder zur Stimulation der Nukleinsäuresynthese.

## Claims

1. Vitamin B₁₂-containing sallow thorn concentrates or extracts.

2. Process for producing a vitamin B₁₂-containing sallow thorn concentrate, characterised in that the liquid and/or volatile components contained in sallow thorn are separated therefrom.

3. Process according to claim 2, characterised in that the liquid and/or volatile components are separated by drying with air, drying with supercritical CO₂, freeze drying, spray drying or concentration under vacuum.

4. Process for producing a vitamin B₁₂-containing sallow thorn extract, characterised in that vitamin B₁₂, optionally together with other ingredients, is extracted from sallow thorn in a known manner.

5. Process according to claim 4, characterised in that continuous or discontinuous liquid/liquid extraction is carried out.

6. Process according to one of claims 2 to 5, characterised in that sallow thorn fruits are used as starting materials.

7. Use of sallow thorn for the manufacture of a pharmaceutical composition for the prophylaxis or treatment of vitamin B₁₂ deficiencies.

8. Use according to claim 7 for the manufacture of a pharmaceutical composition for the treatment of neuropathies and/or erythropoiesis disorders and/or for the stimulation of nucleic acid synthesis.

## Revendications

1. Concentrés ou extraits d'argousier contenant de la vitamine B₁₂.

2. Méthode de fabrication d'un concentré d'argousier contenant de la vitamine B₁₂, caractérisée en ce que les composants liquides et/ou volatils contenus dans l'argousier sont soumis à une séparation.

3. Méthode selon la revendication 2, caractérisée en ce que les composants liquides et/ou volatils sont soumis à une séparation par séchage à l'air, séchage par CO₂ surcritique, lyophilisation, séchage par pulvérisation ou concentration sous vide.

4. Méthode de fabrication d'un concentré d'argousier contenant de la vitamine B₁₂, caractérisée en ce que la vitamine B₁₂, le cas échéant conjointement avec d'autres ingrédients, est extraite de l'argousier selon un procédé connu en soi.

5. Méthode selon la revendication 4, caractérisée en ce que l'on réalise une extraction liquide-liquide continue ou discontinue.

6. Méthode selon l'une des revendications 2 à 5, caractérisée en que l'on utilise des fruits d'argousier comme matières premières.

7. Utilisation de l'argousier pour la fabrication d'un médicament destiné à la prophylaxie ou au traitement de carences en vitamine B₁₂.

8. Utilisation selon la revendication 7 pour la fabrication d'un médicament destiné au traitement des névropathies et/ou troubles de l'érythropoïèse et/ou à la stimulation de la synthèse de l'acide nucléique.
